# EUROPÄISCHE PATENTSCHRIFT

(11) **EP 3 041 353 B1**
(45) Veröffentlichungstag und Bekanntmachung des Hinweises auf die Patenterteilung: **10.10.2018**
(21) Anmeldenummer: 14765882.7
(22) Anmeldetag: 27.08.2014
(51) Int. Cl.: A01N 33/12, A01N 43/40, A01N 47/44, A01N 25/32, A01N 41/04, A61K 31/185, A61K 31/14, A61K 31/155, A61K 31/4425, A61K 31/444

(54) **ZUSAMMENSETZUNGEN UMFASSEND BAKTERISTATIKA UND BRILLANT BLAU G**
COMPOSITIONS COMPRISING BACTERIOSTATIC AGENTS AND BRILLIANT BLUE G
COMPOSITIONS CONTENANT DES AGENTS BACTÉRIOSTATIQUES ET DU BLEU BRILLANT G

(30) Priorität: 02.09.2013 DE 102013109514
(43) Veröffentlichungstag der Anmeldung: 13.07.2016
(73) Patentinhaber: Jacobs University Bremen gGmbH, 28759 Bremen (DE)
(72) Erfinder: GABEL, Detlef, 28357 Bremen (DE); BARTOK, Melinda, 63303 Dreieich (DE)
(74) Vertreter: Weidner Stern Jeschke
(86) Internationale Anmeldenummer: PCT/EP2014/068195
(87) Internationale Veröffentlichungsnummer: WO 2015/028522

(56) Entgegenhaltungen:
- WO-A1-2011/117384
- DE-A1-102005 063 375
- GB-A- 2 403 408
- US-A- 5 767 172
- US-A1- 2009 220 516
- US-B1- 6 583 181
- LIN-HUA JIANG ET AL: "Brilliant Blue G Selectively Blocks ATP-Gated Rat P2X7 Receptors", MOLECULAR PHARMACOLOGY, Bd. 58, 2000, Seiten 82-88, XP002731123,

## Beschreibung

Die Erfindung betrifft Zusammensetzungen, die Bakteriostatika und Brilliant Blau G umfassen.

Wunden auf der Haut und an anderen Körperoberflächen müssen während ihrer Heilung vor bakteriellen Infektionen geschützt werden. Dies geschieht mit unspezifischen Bakteriostatika wie beispielsweise Benzalkoniumchlorid (BAK), Octenidin (Oct), Chlorhexidin (CHex) und ähnlichen Substanzen. Alle diese Substanzen führen aber in den Konzentrationen, in denen sie angewendet werden, auch zu einer Schädigung der Zellen, die zur Schließung der Wunde notwendig sind, z.B. Hautzellen. Dadurch wird der Wundheilungsprozess verzögert.

US 6 583 181 B1 beschreibt, dass der Zusatz eines Elektrolyten wie das Natriumsalz von Ethylendiamintetraessigsäure (EDTA) zu einer desinfizierenden Zusammensetzung, die quartäre Ammoniumverbindungen oder Amine enthält, die Augenreizung verringert und die desinfizierende und sterilisierende Wirksamkeit der Zusammensetzung erhöht.

DE 2005 10 063 375 A1 betrifft zur Wundbehandlung geeignete antimikrobielle Zubereitungen, die Octenidindihydrochlorid verkapselt in Liposomen, beispielsweise Phospholipid-Liposomen, enthalten und eine geringe Zytotoxizität aufweisen.

WO 2011/117384 A1 offenbart eine Wundauflage, umfassend ein polymeres Substrat und eine Zusammensetzung umfassend a) mindestens einen antimikrobiellen Wirkstoff und b) ein die Zytotoxizität absenkendes Mittel, umfassend eine Öl-in-Wasser-Emulsion, die zusätzlich ein oder mehrere Alkandiol(e) und/oder ein oder mehrere Glycerinether aufweist.

US 2009/0220516 A1 beschreibt die Verwendung von Antagonisten des P2X₇-Rezeptors auf Ganglienzellen, beispielsweise Brillantblau G, um Ganglienzellen vor einem Absterben durch zu hohe ATP-Mengen im extrazellulären Raum der Retina zu bewahren.

Jiang et al. 2000 (Jiang LH, Mackenzie AB, North RA, Surprenant A, Brilliant Blue G Selectively Blocks ATP-Gated Rat P2X7 Receptors, Molecular Pharmacology 58 (1) 82-88, DOI: https://doi.org/10.1124/mol.58.1.82) beschreiben, dass Brillantblau G den P2X₇-Rezeptor spezifisch nicht-kompetitiv hemmt.

Aufgabe der vorliegenden Erfindung ist es, eine Möglichkeit bereitzustellen, die Schädigung von an der Wundheilung beteiligten Zellen durch Bakteriostatika zu vermindern.

Zur Lösung der Aufgabe stellt die Erfindung eine Zusammensetzung zur Verwendung als Wunddesinfektionsmittel bereit, wobei die Zusammensetzung mindestens ein Bakteriostatikum und den Farbstoff Brillantblau G, oder ein Salz davon, umfasst, und wobei das Bakteriostatikum ausgewählt ist aus der Gruppe bestehend aus Octenidin, Benzalkoniumchlorid, Chlorhexidin, Cetylpyridiniumchlorid und Polyhexanid, oder Salzen davon, und Kombinationen davon.

Die Erfinder haben überraschend gefunden, dass die zelltoxische Wirkung der oben genannten Bakteriostatika durch die gleichzeitige Gabe des Farbstoffs Brillantblau G (BBG) stark reduziert wird. Gleichzeitig bleibt deren bakteriostatische Wirkung weitgehend erhalten, so dass ein Schutz vor Bakterien immer noch gegeben ist. Die Erfindung macht sich somit den überraschenden Befund zunutze, dass diese Bakteriostatika in Gegenwart von BBG somit weiterhin ihre vor bakteriellen Infektionen schützende Wirkung ausüben, während ihre toxische Wirkung auf menschliche und nicht-menschliche Zellen, insbesondere Säugetierzellen, deutlich reduziert ist. Diese Wirkung ist spezifisch für BBG, denn für den strukturell sehr ähnlichen Farbstoff BBR (Brillantblau R) zeigte sich diese Schutzwirkung nicht.

Unter "Brillantblau G" (Coomassie-Brillant-Blau G-250, Acid Blue 90) wird eine Verbindung der nachfolgenden Formel (I) verstanden:

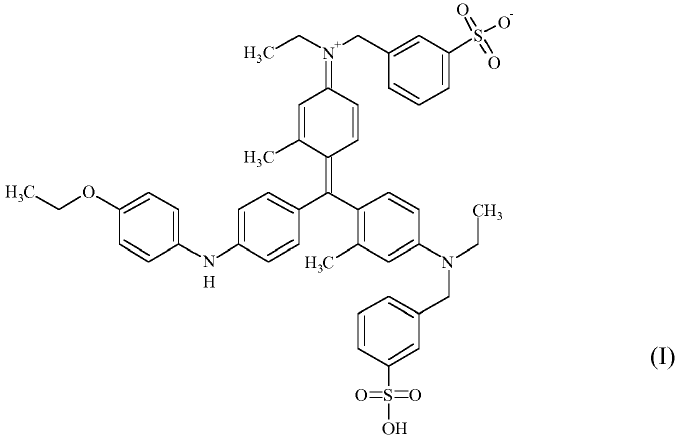

Salze von BBG sind bevorzugt pharmazeutisch annehmbare Salze. Ein Beispiel für ein geeignetes Salz ist ein Natriumsalz.

Vorzugsweise ist Brillantblau G in der Zusammensetzung zur Verwendung als Wunddesinfektionsmittel in einer Menge vorhanden, die wirksam ist, um die Toxizität des Bakteriostatikums für menschliche und nicht-menschliche Säugetierzellen zu vermindern. Vorzugsweise beträgt der Anteil an Brillantblau G in der Zusammensetzung zur Verwendung als Wunddesinfektionsmittel ≥0,003 Gew.-% und <0,25 Gew.-%, bezogen auf das Gesamtgewicht der Zusammensetzung. Besonders bevorzugt beträgt der Anteil an Brillantblau G in der Zusammensetzung zur Verwendung als Wunddesinfektionsmittel 0,003-0,2 Gew. %, bevorzugt 0,003-0,1 Gew. %, weiter bevorzugt 0,003-0,08 Gew. % oder 0,003-0,05 Gew. %, besonders bevorzugt 0,005-0,03 Gew. %, bezogen auf das Gesamtgewicht der Zusammensetzung.

Der Anteil an Brillantblau G (BGG) in der Zusammensetzung zur Verwendung als Wunddesinfektionsmittel wird vom Fachmann gegebenenfalls von der Konzentration und/oder der Art des(der) verwendeten Bakteriostatikums (Bakteriostatika) gewählt. Bei höheren Konzentrationen des Bakteriostatikums, oder im Falle mehrerer Bakteriostatika der Gesamtkonzentration der Bakteriostatika wird der Fachmann tendenziell den Anteil an BGG erhöhen. Geeignete Verhältnisse können leicht mittels Tests an Zellen, z.B. HCE-Zellen, ermittelt werden.

Bereichsangaben wie beispielsweise 0,003-0,2 Gew. % sind hier so zu verstehen, dass jeder beliebige Zwischenwert zwischen den Grenzwerten des jeweiligen Bereichs und auch beliebige Bereiche innerhalb des Bereichs ausdrücklich mit gemeint ist. Im Fall einer Angabe, die nur Ganzzahlen betreffen kann, wie beispielsweise eine Zahl von C-Atomen, bedeutet dies selbstverständlich, dass nur Ganzzahlen gemeint sind. Eine Bereichsangabe wie 0,003-0,2 Gew. % umfasst daher beispielsweise einen Einzelwert wie 0,025 Gew.-% oder auch Bereiche wie 0,003-0,025 Gew.-% oder 0,010-0,15 Gew.-%.

Unter einem "Bakteriostatikum" wird eine Verbindung verstanden, die das Wachstum von Bakterien hemmt, wobei der Begriff auch Verbindungen umfasst, die bei geeigneter Konzentation eine abtötende Wirkung auf Bakterien haben können. Beispiele für Bakteriostatika sind Octenidin, Benzalkoniumchlorid, Chlorhexidin, Cetylpyridiniumchlorid und Polyhexanid, oder Salze davon.

Unter "Octenidin" wird eine Verbindung der folgenden Formel (II) 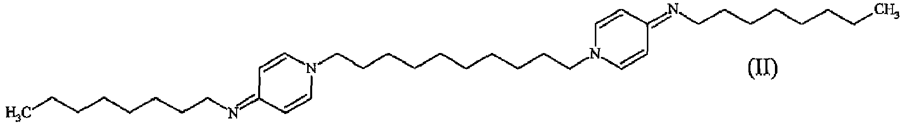 verstanden.

Unter Benzalkoniumchlorid wird hier eine Verbindung der Formel (III) 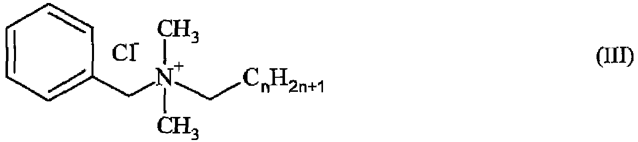 verstanden, wobei n = 8, 10, 12, 14, 16 oder 18 sein kann.

Unter "Chlorhexidin" wird hier eine Verbindung der Formel (IV) 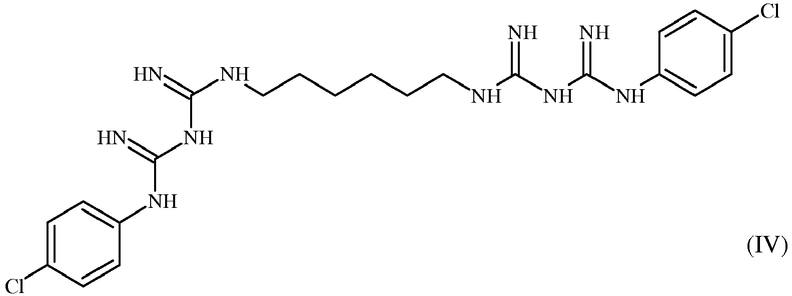 verstanden.

Unter Cetylpyridiniumchlorid wird hier eine Verbindung der allgemeinen Formel (V) 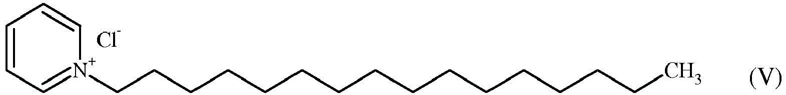 verstanden.

Unter "Polyhexanid" (Polyhexamethylenbiguanid, PHMB) wird hier eine Verbindung der allgemeinen Formel (VI) 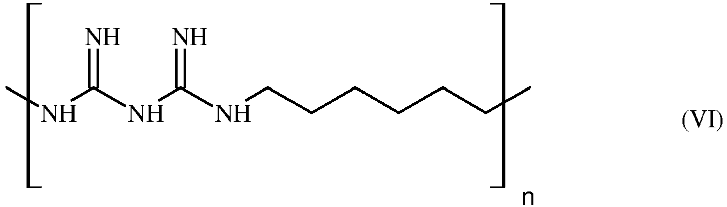 verstanden, wobei n = 2-15 oder im Durchschnitt 5 ist.
Im folgenden wird die Erfindung anhand von Ausführungsbeispielen näher erläutert.

Die Erfindung wird im Folgenden rein zu Veranschaulichungszwecken anhand der angehängten Figuren und von Ausführungsbeispielen näher erläutert.
Figur 1 Effekt von 0,025% BBG auf die Toxizität von unterschiedlichen Konzentrationen (in Gew.-%) von Benzalkoniumchlorid (BAK) auf HCE-Zellen (HCE = human corneal epithelium, Menschliche Hornhautepithelzellen) bei verschiedenen Einwirkdauern (5, 30, 60 min). Oben: BAK ohne BBG. Unten: BAK mit BBG. Inkubationsmedium: Phosphat-gepufferte Kochsalzlösung (PBS).
Figur 2 Effekt von BBG (in Gew.-%) in unterschiedlichen Konzentrationen auf die Toxizität von BAK bei 0,003% (oben) und 0,008% (unten). PBS = Phosphat-gepufferte Kochsalzlösung; BBS = balanced salt solution (ausgeglichene Salzlösung).
Figur 3 Effekt von BBG (0,025 Gew.-%) auf die Toxizität von Octenidindihydrochlorid (Oct) in unterschiedlichen Konzentrationen (in Gew.-%) nach 5 min Inkubation (oben) und 30 min Inkubation (unten). Octenidin alone = Octenidin allein (ohne BBG).
Figur 4 Effekt von BBG (0,025 %) auf die Toxizität von Cetylpyridiniumchlorid (CPC) in unterschiedlichen Konzentrationen (in Gew.-%) nach 5 min Inkubation (oben) und 30 min Inkubation (unten). CPC alone = Cetylpyridiniumchlorid allein (ohne BBG).
Figur 5 Effekt von BBG (0,025 %) auf die Toxizität von Chlorhexidin (CHex) in Form von Chlorhexidindigluconat in unterschiedlichen Konzentrationen (in Gew.-%) nach 5 min Inkubation (oben) und 30 min Inkubation (unten). Chlorhexidin alone = Chlorhexidin allein (ohne BBG)

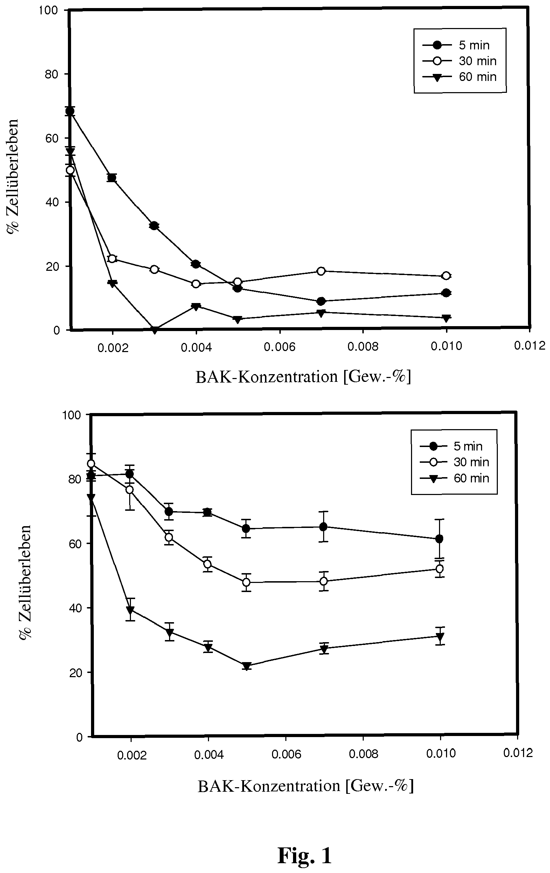

Figur 1 zeigt die Wirkung von BBG 0,025% Gew.-% auf die Toxizität von unterschiedlichen Konzentrationen (in Gew.-%) von Benzalkoniumchlorid (BAK) auf HCE-Zellen (HCE = human corneal epithelium, Menschliche Hornhautepithelzellen) bei verschiedenen Einwirkdauern (5, 30, 60 min). Die Schutzwirkung (=erhöhtes Zellüberleben) ist selbst bei 60 min Einwirkzeit noch deutlich vorhanden.

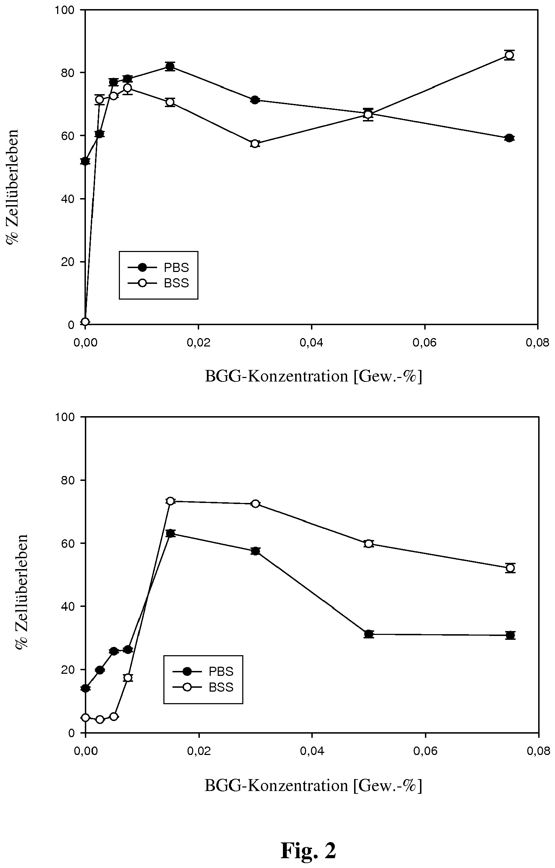

Figur 2 zeigt die Wirkung von BBG (in Gew.-%) in unterschiedlichen Konzentrationen und zwei unterschiedlichen Medien (PBS und BSS) auf die Toxizität von BAK bei 0,003% (oben) und 0,008% (unten) bei HCE-Zellen. Die Schutzwirkung von BBG bei niedrigeren BAK-Konzentrationen setzt schon bei 0,003% BBG ein. Bei höheren BAK-Konzentrationen sind 0,015% notwendig. Noch höhere Konzentrationen (0,05% und höher) führen zu einer leichten Erniedrigung des Überlebens.

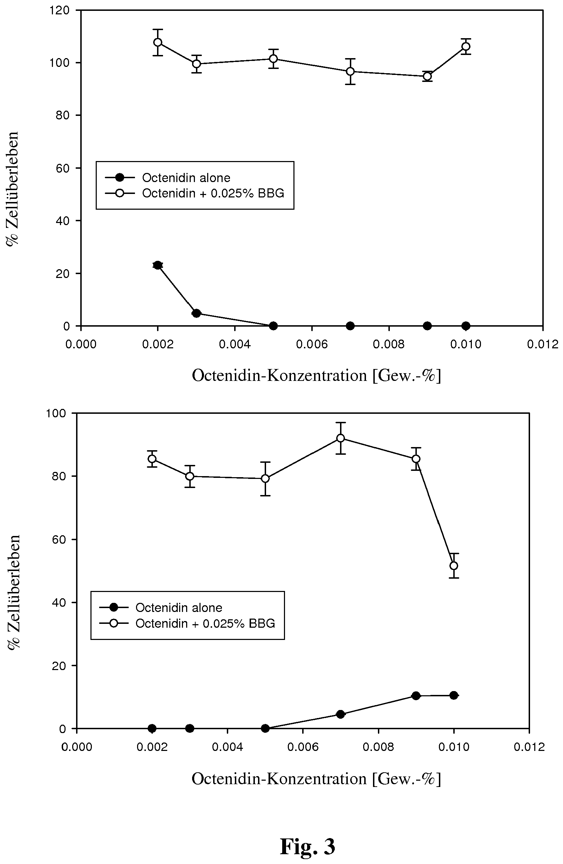

Figur 3 zeigt die Wirkung von BBG (0,025 Gew.-%) auf die Toxizität von Octenidin (Oct) Form von Octenidindihydrochlorid in unterschiedlichen Konzentrationen (in Gew.-%) nach 5 min Inkubation (oben) und 30 min Inkubation (unten). Octenidin alone = Octenidin allein (ohne BBG). Octenidin ist schon bei 0,002 Gew.-% und 5 min Einwirkzeit stark zellschädigend. BBG (0,025 Gew.-%) kann diesen Effekt bei 5 min Inkubation (links) bis zu einer Oct-Konzentration von 0,01% (der höchsten getesteten Konzentration) verhindern. Bei 30 min Einwirkzeit (rechts) ist erst ab 0,009 Gew.-% Oct der Schutz nicht mehr maximal.

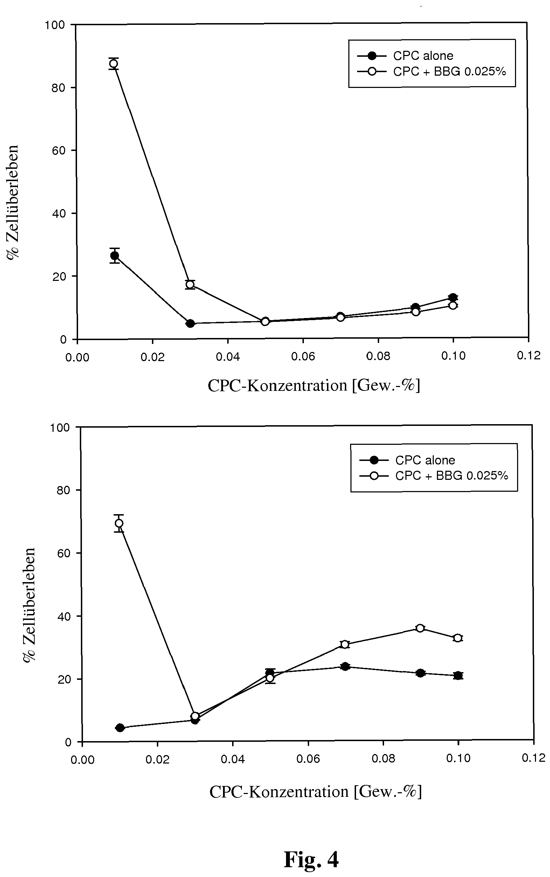

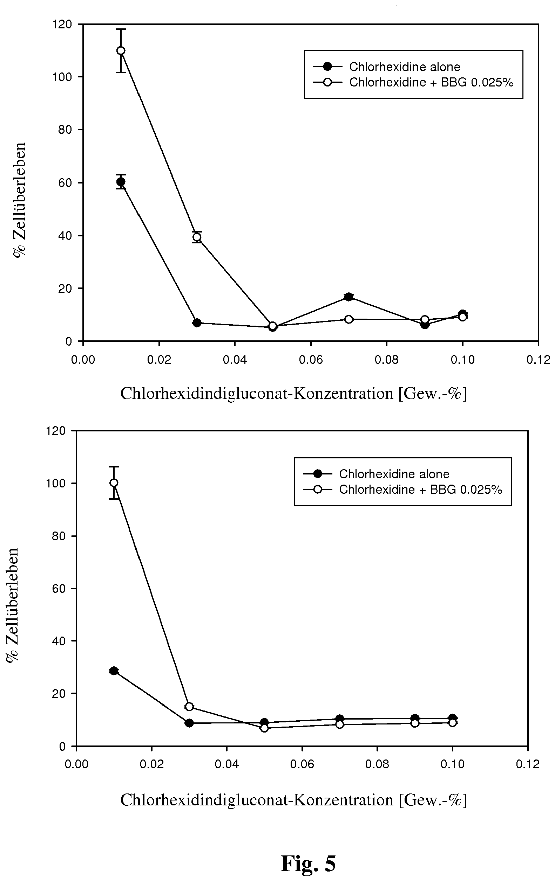

Die Figuren 4 und 5 zeigen die Wirkung von BBG (0,025 Gew.-%) auf die Toxizität von Cetylpyridiniumchlorid (CPC, Fig. 4) und Chlorhexidin (CHex in Form von Chlorhexidindigluconat, Fig. 5) in unterschiedlichen Konzentrationen (in Gew.-%) nach 5 min Inkubation (jeweils oben) und 30 min Inkubation (jeweils unten). Mit CPC ist der Schutzeffekt nur bis etwa 0,02% CPC gegeben, dies jedoch auch noch bei 30 min Einwirkzeit (Fig. 4, unten). Ähnliches gilt für Chlorhexidin.

Zur Untersuchung des Einflusses von BBG auf die bakteriostatische Wirkung wurden Bakterien 24 h mit erfindungsgemäßen Zusammensetzungen, Benzalkoniumchlorid (BAK) und Octenidin (Oct) als Bakteriostatikum enthielten, inkubiert. Wenn sich die Inkubationsmischung trübte, wurde das als Bakterienwachstum interpretiert. Zum Vergleich wurde Brillantblau R (BBR) anstelle von BBG eingesetzt. Die Ergebnisse sind in den Tabellen 1-4 wiedergegeben.

**Tabelle 1: Einfluss von BBG und BBR auf die bakteriostatische Wirkung von BAK gegenüber Escherichia coli (E. coli), + = Bakterienwachstum nach 24 h Inkubation, - = kein Bakterienwachstum nach 24 h Inkubation, R = Brillantblau R (BBR), G = Brillantblau G (BBG).**

| BAK-Konzentration (Gew.-%) | | | | | | | |
|---|---|---|---|---|---|---|---|
| | 0,01% | 0,006% | 0,004% | 0,0025% | 0,0016% | 0,001 % | Kein BAK |
| 0,030% R | - | + | + | + | + | + | + |
| 0,030% G | + | + | + | + | + | + | + |
| 0,015% R | - | - | - | + | + | + | |
| 0,015% G | - | - | + | + | + | + | |
| 0,007% R | - | - | - | - | + | + | |
| 0,007% G | - | - | - | + | + | + | |
| BAK | - | | | | | - | |

**Tabelle 2: Einfluss von BBG und BBR auf die bakteriostatische Wirkung von BAK gegenüber Bacillus sp., + = Bakterienwachstum nach 24 h Inkubation, - = kein Bakterienwachstum nach 24 h Inkubation, R = Brillantblau R (BBR), G = Brillantblau G (BBG).**

| BAK-Konzentration (Gew.-%) | | | |
|---|---|---|---|
| | 0.01% | 0.001% | Kein BAK |
| 0.007% R | - | - | |
| 0.007% G | - | - | |
| 0.030% R | | | + |
| 0.030% G | | | + |
| BAK | | - | |

BBR und BBG beeinflussen das Bakterienwachstum nicht (rechte Spalten). Gram-negative Bakterien (hier E. coli) sind widerstandsfähiger als Gram-positive Bakterien (hier Bacillus sp.) und wachsen konzentrationsabhängig. BBG hat dabei einen etwas größeren Einfluss als BBR, aber auch BBR hebt einen Teil der bakteriostatischen Wirkung auf.

**Tabelle 3: Einfluss von BBG auf die bakteriostatische Wirkung von Octenidin (Oct) gegenüber Gram-negativen Bakterien., + = Bakterienwachstum nach 24 h Inkubation, - = kein Bakterienwachstum nach 24 h Inkubation, BBG = Brillantblau G.**

| Octenidin-Konzentration (Gew.-%) | | | | | | | |
|---|---|---|---|---|---|---|---|
| | 0,01% | 0,009% | 0,007% | 0,005% | 0,003% | 0,002% | 0% |
| Escherichia coli DH5α | | | | | | | |
| 0,030% BBG | + | + | + | + | + | + | + |
| 0,015% BBG | - | - | - | + | + | + | + |
| 0,007% BBG | - | - | - | - | - | + | + |
| Octenidin | - | - | - | - | - | - | |

| Pseudomonas putida DSM 291 | | | | | | | |
|---|---|---|---|---|---|---|---|
| 0,030% BBG | + | + | + | + | + | + | + |
| 0,015% BBG | - | - | - | + | + | + | + |
| 0,007% BBG | - | - | - | - | - | + | + |
| Octenidin | - | - | - | - | - | - | |

| Vibrio sp. Gal12 | | | | | | | |
|---|---|---|---|---|---|---|---|
| 0,030% BBG | + | + | + | + | + | + | + |
| 0,015% BBG | - | - | - | + | + | + | + |
| 0,007% BBG | - | - | - | - | - | + | + |
| Octenidin | - | - | - | - | - | - | |

**Tabelle 4: Einfluss von BBG auf die bakteriostatische Wirkung von Octenidin (Oct) gegenüber Gram-positiven Bakterien., + = Bakterienwachstum nach 24 h Inkubation, - = kein Bakterienwachstum nach 24 h Inkubation, +/- = geringes Wachstum nach 24 h Inkubation, BBG = Brillantblau G.**

| Octenidin-Konzentration (Gew.-%) | | | | | | | |
|---|---|---|---|---|---|---|---|
| | 0,01% | 0,009% | 0,007% | 0,005% | 0,003% | 0,002% | 0% |
| Bacillus subtilis | | | | | | | |
| 0,030% BBG | - | - | + | + | + | + | + |
| 0,015% BBG | - | - | - | - | + | + | + |
| 0,007% BBG | - | - | - | - | - | + | + |
| Octenidin | - | - | - | - | - | - | |

| Bacillus megaterium | | | | | | | |
|---|---|---|---|---|---|---|---|
| 0,030% BBG | - | - | +/- | +/- | + | + | + |
| 0,015% BBG | - | - | - | - | +/- | + | + |
| 0,007% BBG | - | - | - | - | - | - | + |
| Octenidin | - | - | - | - | - | - | |

| Clavibacter michiganensis | | | | | | | |
|---|---|---|---|---|---|---|---|
| 0,030% BBG | - | - | + | + | + | + | + |
| 0,015% BBG | - | - | - | - | + | + | + |
| 0,007% BBG | - | - | - | - | - | + | + |
| Octenidin | - | - | - | - | - | - | |

| Paenibacillus sp. | | | | | | | |
|---|---|---|---|---|---|---|---|
| 0,030% BBG | - | + | + | + | + | + | + |
| 0,015% BBG | - | - | - | - | + | + | + |
| 0,007% BBG | - | - | - | - | - | - | + |
| Octenidin | - | - | - | - | - | - | |

Die Resultate mit Octenidin entsprechen bei Gram-negativen Bakterien in etwa denen mit BAK. Bei Gram-positiven Bakterien wurde die bakteriostatische Wirkung von Octenidin zum Teil wieder aufgehoben, so dass höhere Octenidin-Konzentrationen notwendig waren, um denselben bakteriostatischen Effekt zu erreichen.

## Patentansprüche

1. Zusammensetzung umfassend mindestens ein Bakteriostatikum und den Farbstoff Brillantblau G gemäß nachfolgender Formel (I) 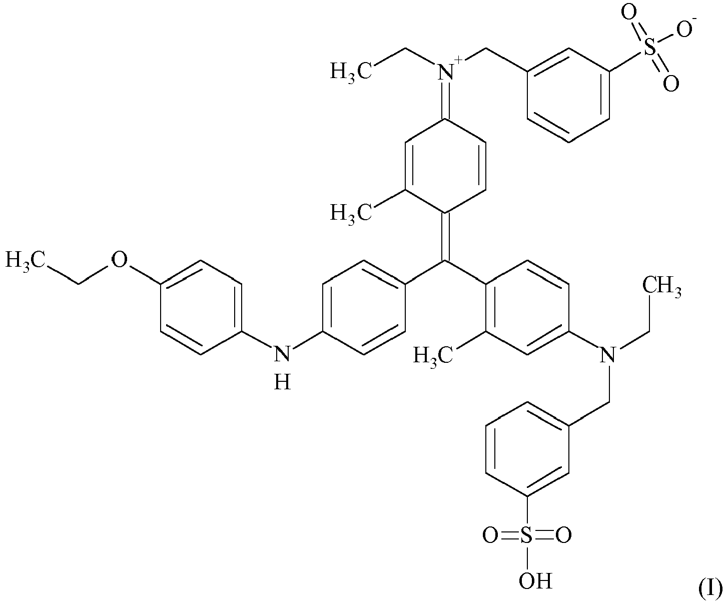 oder ein Salz davon, zur Verwendung als Wunddesinfektionsmittel, wobei das Bakteriostatikum ausgewählt ist aus der Gruppe bestehend aus Octenidin, Benzalkoniumchlorid, Chlorhexidin, Cetylpyridiniumchlorid und Polyhexanid, oder Salzen davon, und Kombinationen davon.

2. Zusammensetzung zur Verwendung als Wunddesinfektionsmittel nach Anspruch 1, wobei der Anteil an Brillantblau G in der Zusammensetzung ≥0,003 Gew.-% und <0,25 Gew.-%, bezogen auf das Gesamtgewicht der Zusammensetzung, beträgt.

3. Zusammensetzung zur Verwendung als Wunddesinfektionsmittel nach Anspruch 2, wobei der Anteil an Brillantblau G in der Zusammensetzung 0,003-0,2 Gew. %, bevorzugt 0,003-0,1 Gew. %, weiter bevorzugt 0,003-0,08 Gew. % oder 0,003-0,05 Gew. %, besonders bevorzugt 0,005-0,03 Gew. %, bezogen auf das Gesamtgewicht der Zusammensetzung, beträgt.

## Claims

1. A composition comprising at least one bacteriostatic agent and the dye Brilliant Blue G according to the following formula (I) 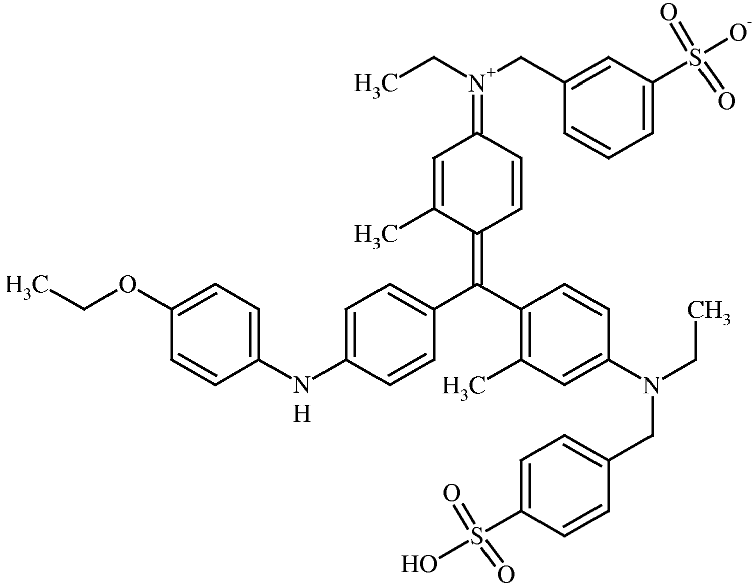 or a salt thereof, for use as a wound disinfectant, wherein the bacteriostatic agent is selected from the group consisting of octenidine, benzalkonium chloride, chlorhexidine, cetylpyridinium chloride and polyhexanide or salts thereof and combinations thereof.

2. The composition for use as a wound disinfectant according to claim 1, wherein the amount of Brilliant blue G in the composition is ≥ 0.003 wt.% and < 0.25 wt.% with respect to the total weight of the composition.

3. The composition for use as a disinfectant according to claim 2, wherein the amount of Brilliant blue G in the composition is 0.003 - 0.2 wt.%, preferably 0.003 - 0.1 wt.%, more preferably 0.003 - 0.08 wt.% or 0.003-0.05 wt.%, particularly preferably 0.005 - 0.03 wt.% with respect to the total weight of the composition.

## Revendications

1. Composition comprenant au moins un agent bactériostatique et le colorant bleu brillant G selon la formule suivante (I) 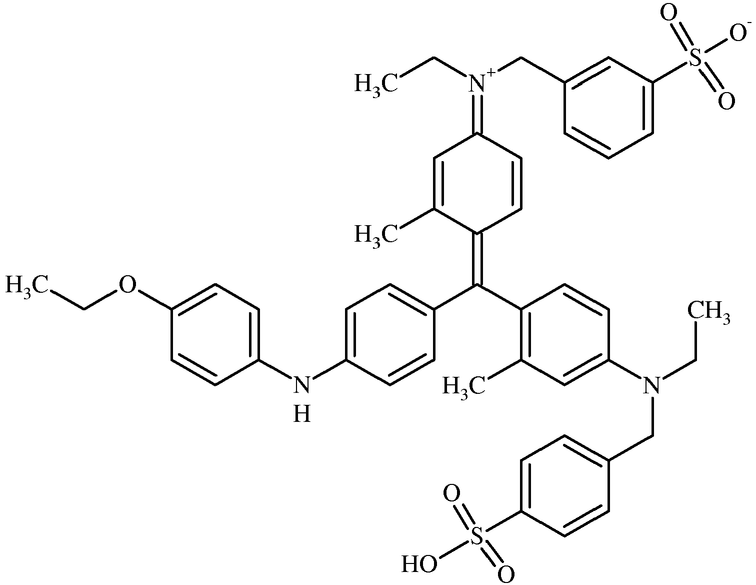 ou un de ses sels, pour une utilisation en tant que désinfectant de plaie, où l'agent bactériostatique est sélectionné parmi le groupe comprenant l'octénidine, le chlorure de benzalkonium, la chlorhexidine, le chlorure de cétylpyridinium et le polyhexanide ou leurs sels et des combinaisons de ceux-ci.

2. Composition pour une utilisation en tant que désinfectant de plaie selon la revendication 1, où la proportion de bleu brillant G dans la composition est ≥ 0,003 % en poids et < 0,25 % en poids par rapport au poids total de la composition.

3. Composition pour une utilisation en tant que désinfectant de plaie selon la revendication 2, où la proportion de bleu brillant G dans la composition est comprise entre 0,003 et 0,2 % en poids, de préférence entre 0,003 et 0,1 % en poids, plus préférablement entre 0,003 et 0,08 % en poids ou 0,003 - 0,05 % en poids, de façon particulièrement préférée 0,005 - 0,03 % en poids par rapport au poids total de la composition.
